# EUROPEAN PATENT SPECIFICATION

(11) **EP 0 552 504 B1**
(45) Date of publication and mention of the grant of the patent: **29.04.1998**
(21) Application number: 92200159.9
(22) Date of filing: 20.01.1992
(51) Int. Cl.: C07D 223/10, A61K 31/55, A61K 47/00

(54) **Pharmaceutical compositions containing 1-oleylazacycloheptan-2-one as enhancing agent of transport across biological membranes**
1-Oleoylazacycloheptanon(2) enthaltende Arzneimittel zur Verstärkung des Transports durch biologische Membranen
Composés pharmaceutiques contenant 1-oléoylazacycloheptan-one-2 comme agent pour renforcer le transport par des membranes biologiques

(43) Date of publication of application: 28.07.1993
(73) Proprietor: SCHWARZ PHARMA AG, 40789 Monheim (DE); RIJKSUNIVERSITEIT LEIDEN, 2300 RA Leiden (NL)
(72) Inventor: Boddé, Henry Ernest Center for Biopharmac.Sciences, NL-2300 RA Leiden (NL); Bouwstra, Johannes Aaltje, NL 2300-RA Leiden (NL); Ponec, Maria Helène, Dept.of Dermatology, NL 2333-AA Leiden (NL); Spies, Ferdinand, Dept. of Electron Microscopy, NL 2333-AA Leiden (NL); Sandrock, Klaus, D-4018 Langenfeld (DE)
(74) Representative: de Bruijn, Leendert C.

(56) References cited:
- EP-A- 0 060 970
- EP-A- 0 129 284
- EP-A- 0 160 111
- EP-A- 0 251 425
- EP-A- 0 268 218
- WO-A-87/06583
- WO-A-89/09800
- DE-A- 2 616 993
- GB-A- 1 343 426
- US-A- 4 310 525
- US-A- 4 879 275
- J. APPL. POLYM. SCI. vol. 11, no. 7, 1967, pages 1299 - 1313; SCELIA R. P. ET. AL.: 'Some Effects of Cocatalyst Structure on the Anionic Polymerisation of e-Caprolactam.'

## Description

### Background of the invention

### 1. Field of invention

The invention relates in general to pharmaceutical compositions containing 1-oleylazacycloheptan-2-one in the function of human or animal epithelial membrane permeability enhancer. Furthermore the invention encompasses the use of above pharmaceutical compositions for the treatment of for instance inflammation as well as the compound per se.

### 2. Background of the prior art

One of the epithelial membranes of interest is the skin. More in particular the skin is frequently used as a portal of entry into the body for two classes of physiologically active agents: (I) systemically active agents, (II) locally active agents. For an increasing number of systemically active agents, delivery through the skin is favourable, since absorption kinetics can be controlled, and various side effects (such as hepatic first-pass metabolism) avoided.

For locally active agents, such as anti-inflammatory drugs, the skin is both the portal of entry and the target organ.

Generally, the greatest problem in applying physiologically active agents topically is that the skin is an effective barrier for penetration of such agents. The epidermis of the skin has an exterior layer of dead cells called the "stratum corneum" which is tightly structured, lipid rich and which provides an effective barrier against all kinds of agents whether used alone or in water or oil solution or suspension. However, once a physiologically active agent has penetrated the stratum corneum it can readily pass through the basal layer of the epidermis and into the dermis.

In the prior art many vehicle compositions are disclosed containing 1-substituted azacycloheptan-2-ones useful in enhancing penetration of the skin by physiologically active agents.

In British patent specification 1,553,309 a large number of 1-al-kylazacycloheptan-2-ones, in particular 1-n-dodecylazacycloheptan-2-one are disclosed. As indicated on page 1 of this British patent these compounds are found to be useful as vehicles for delivering physiologically active agents through body membranes such as the skin and for retaining these agents in body tissues. However, no details concerning the possible mode(s) of action of these 1-C₁-₁₈ alkylazacycloheptan-2-ones are reported in this patent.

US-A-3,989,816 discloses a method for enhancing the penetration of physiologically active agents through human and animal membranes e.g. the skin by administering to a human or animal membrane a composition comprising an effective amount of the active agent and a suitable amount of an 1-alkyl-azacycloheptan-2-one, the alkyl substituent having the formula -(CH₂)ₙ-R, wherein R is selected from the group consisting of alkyl moieties having 1-18 carbon atoms and aryl groups, and n is 0 or a positive integer from 1-10. Preferably the alkyl substituent has 1-12 carbon atoms. However, the penetration enhancing properties of these compounds are considered inadequate.

Further US-A-4,310,525 relates to the application of an effective amount of 1-dodecyl-azsacycloheptan-2-one as the sole anti-inflammatory agent for treating inflammation in humans and animals. The active compound is used in the form of a composition applied topically on the human or animal skin. The concentration of 1-dodecylazacycloheptan-2-one in the composition may vary from about 10 to 100 % and preferably from about 42 to 80 % by weight. According to this US-A-4,310,525 the compound 1-dodecyl-azacycloheptan-2-one per se is not used as penetration enhancer for physiologically active agents, but is, apparently, as such capable of temporarily reducing the signs and symptoms of inflammation.

EP-A-0 129 284 relates to topical pharmaceutical compositions containing a physiologically active agent and a binary combination of 1-dodecyl-azacycloheptan-2-one on the one hand and a specific C₃-C₄-diol or a 1-substituted azacycloalkyl-2-one on the other hand as penetration enhancing agents. However, the use of a mixture of specific membrane-penetration enhancing agents at specific ratio's is considered undesirable whereas nothing is disclosed about the possible toxic properties of these enhancer mixtures.

EP-A-0 251 425 discloses an article for the administration of physiologically active substances comprising a plasticized polyvinylchloride layer containing from about 20 to about 70 % by wt. of a polyvinylchloride resin from about 20 to about 70 % of a plasticizer and from about 0.5 to 35 % of the physiologically active substance. The plasticizer may be 1-dodecyl-azacycloheptan-2-one which in fact also acts as a membrane penetration enhancer for the physiologically active compound through the stratum corneum.

### Summary of the invention

Surprisingly, it has been found by Applicant that the compound 1-oleylazacycloheptan-2-one as such is both a more potent enhancer for percutaneous absorption than the 1-alkylazacycloheptan-2-ones according to the prior art and less toxic than the 1-dodecylazacycloheptan-2-one, considered the preferred compound according to the above cited prior art.

Therefore, the invention relates to 1-oleylazacycloheptan-2-one per se, to compositions comprising at least one physiologically active agent and the penetration enhancer according to the invention, to devices such as skin patches containing such a composition applicable for enhancing the penetration of active agents through human and animal epithelial membranes while applying the specific 1-alkyl-azacycloheptan-2-one derivative mentioned above.

### Brief description of the drawings

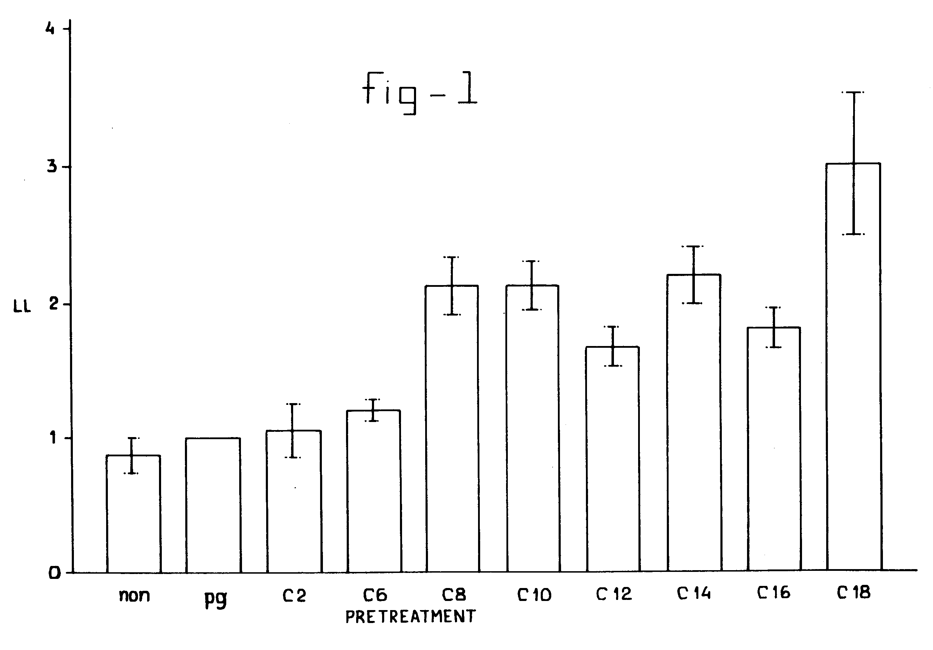

Figure 1 is a graph showing the influence of the length of the alkyl chain of the 1-alkylazacycloheptan-2-ones on percutaneous nitroglycerine transport enhancement (see test 1).

Figure 2 is a graph showing the influence of the length of the alkyl chain of the 1-alkylazacycloheptan-2-ones on the thermal behaviour of human stratum corneum.

Figure 3 is a graph showing the IC50-values and enhancement factors of the 1-alkylazacycloheptan-2-ones tested in relationship to the length of the alkyl chain in these compounds.

Figure 4 is an electron microscopic picture of the stratum corneum subjected to a freeze-fracture replication procedure according to test 3 in the presence of 1-oleylazacycloheptan-2-one.

### Detailed description of the invention

The compound described above is useful in relationship to percutaneous penetration i.e. the compound according to the invention enhances the percutaneous absorption of topically administered physiologically active agents, e.g. drugs, growth factors, radical scavengers, etc.

The compound according to the invention may be prepared, for example, by treating azacycloheptan-2-one with oleyl halide or preferably a relevant hydrocarbyl residue with an excellent nucleophilic leaving group, for example mesylate, tosylate, etc. in the presence of a base in an inert atmosphere. As a base sodium hydride may be used. The reaction is carried out under anhydrous conditions in e.g. a hydrocarbon solvent like dry toluene at room temperature or higher for a period of 1 or more hours. In this respect, it is emphasized that the compound according to the invention may also be prepared according to other methods known from the prior art for similar compounds.

The concentration of 1-oleylazacycloheptan-2-one, which may be used in the present invention, is an effective concentration for enhancing percutaneous absorption of active agents. Generally, this concentration ranges from 1 to about 95, and preferably from 5 to about 40 % by wt. of the composition.

The compositions according to the invention comprise at least one physiologically active agent, e.g. an antibacterial agent, an antifungal agent, an active steroid, an antihistaminic agent, antihypertensives and other cardiovascular drugs, sex hormones, anti-diabetics, hypnotics, anti-parkinson drugs, local anaesthetics, anticoagulants, analgesics, antimigraine drugs. The physiologically active agents may be used at a safe and effective level in the compositions according to the invention, being in general from about 0.01 % to about 30 % by weight of the composition. However, levels outside this range may also be used.

Examples of antibacterial agents which may be used in this invention include penicillins, cephalosporins, penicillinase, erythromycins, tetracyclines, chloramphenicols, streptomycins, etc.

Examples of antifungal agents i.e. fungistatic and fungicidal agents are thiabendazole, chloroxine, amphotericin, candicidin, fungimycin, etc.

Examples of steroids which may be used with the 1-oleylazacycloheptan-2-one include corticosteroids such as e.g. cortisone, cortodoxone, prednisone, etc. Examples of retinoids such as retinoic acid may also be used.

Dosage forms for topical application may include solutions, nasal sprays, lotions, ointments, cremes, gels, suppositories, sprays, aerosols as well as devices such as skin patches, bandages and dressings containing a composition according to the invention. Typical conventional pharmaceutical carriers which make up the foregoing dosage forms include water, acetone, isopropylalcohol, ethylalcohol, polyvinylpyrrolidone, propylene glycol, fragrances, gel-producing materials, mineral oil, stearyl alcohol, steric acid, spermaceti, sorbitan monoleate, "Polysorbates", "Tweens", etc.

The amount of the composition and thus of the physiologically active agent therein, to be administered will obviously be an effective amount for the desired result expected therefrom. This, of course, will be ascertained by the ordinary skill of the practitioner. Due to enhanced activity which is achieved with the enhancer, the dosage of the active agent may often be decreased from that generally applicable.

As used herein the term "topical" is intended to include application to all external membrane barriers including the cutaneous or epidermis regions and the mucous membranes including the gastrointestinal tract, the respiratory tract and the genitourinary tract.

### EXPERIMENTAL

The potent membrane enhancer properties of 1-oleylazacycloheptan-2-on may be derived from a series of different experiments according to which the compound according to the invention is compared to 1-alkylazacyclopheptan-2-ones known from the prior art.

The series of different experiments comprise four tests:
1. Skin transport kinetics;
2. Differential thermal analysis;
3. Freeze-fracture electron microscopy; and
4. In vitro skin cell toxicity screening, i.e. the collagen contraction test.

### Test 1.

### Skin transport kinetics

Human stratum corneum samples (18 mm in diameter) were obtained from fresh human skin (abdomen) by trypsinization. The stratum corneum samples were firstly prehydrated at 95 % relative humidity (RH) over a saturated disodium hydrogen phosphate solution, then either submerged for 24 hours at 34°C with 200 microliter of a 10 % solution of an alkyl-azacycloheptan-one in propylene glycol by topical application, or directly used for a diffusion study.

The stratum corneum samples were subsequently sandwiched between silastic sheetings (127 micrometer thick silicone membranes, Dow Corning) which have been proven not to control the transport rate of the model drug used in the study, nitroglycerin. The thus obtained sandwiches were clamped in two-chamber flow-through diffusion cells and subjected to 24 hours diffusion experiments. The donor compartment having a volume of 16 µl was flushed with a 0.05 % nitroglycerin solution at a pumping rate of 35 ml/hr. A saturated disodium hydrogen phosphate solution was pumped at a rate of 5 ml/hr through the acceptor chamber having a volume of 16 µl. Fractions of the acceptor fluid were collected at 1 hour intervals, and nitroglycerin was detected using a reversed phase HPLC procedure (chromsphere C₁₈ 100x30 mm column eluted at 22°C (methanol:water = 1:1) at a 1.1 ml/min flow rate (Spectroflow 400, Kratos.). The absorbance at 201 nm of the effluent was monitored on Waters 450 var. wavelength detector).

### Test 2.

### Differential thermal analysis (DTA)

For differential thermal analysis human stratum corneum samples were pretreated (after prehydration) by submersion in the in test 1 described enhancer solutions for 24 hours, using untreated prehydrated stratum corneum samples as controls. Differential thermal analysis of ca. 20 mg samples was then performed on a differential scanning calorimeter (Maple).

### Test 3.

### Freeze-fracture electron microscopy

For freeze-fracture electron microscopy stratum corneum samples were pretreated during 24 hours with solutions of azacycloheptanones in propylene glycol by applying about 200 microliter per cm² of the solution on the apical surface. Samples were then snapfrozen in melting nitrogen (method according to Umrath, J.Microsc. 101: 103 (1974)), cross-fractured (Holman et al., J.Invest. Dermatol, 94: 332 (1990) )in a Balzers Freeze Fracture apparatus (Balzers 400 BAF) at high vacuum. The fracture surfaces were then shadowed with Pt and overcast with a layer of carbon. Upon thawing, the samples were cleaned to obtain the surface replicas. These were scooped up on copper grids and examined in a transmission electron microscope (Philips 401, operated at 80 kV).

### Test 4.

### Collagen contraction test

Skin toxicity tests were performed using the so-called collagen contraction test (Ponec et al. Journal of Pharmaceutical Sciences Vol. 78, No. 9, September 1989 and Vol. 79, No. 4, April 1990). In this test collagen sheets containing human dermal fibroblasts are subjected to a challenge by toxic agents (in this case the 1-alkylazacycloheptan-2-ones) and the resulting inhibition of the contraction, normally induced by the collagenase released by the fibroblasts, is measured as a function of time. The concentration of the agent, at which the shrinkage is 50 % of the control, is taken as the IC50, the "50 % inhibition level". The outcome of this test proved to be highly comparable to both fibroblast- and human epidermal keratinocyte growth inhibition tests.

### RESULTS

Re test 1:
From the kinetical results summarized in table 1 below it is quite clear that the oleyl derivative is by far the most potent enhancer with respect to nitroglycerin transport.

**Table 1**

| Effects of alkyl chainlength of azone derivatives (1-alkylazacycloheptan-2-one derivatives) on the penetration of nitroglycerine through stratum corneum at 33°C. | | | | | |
|---|---|---|---|---|---|
| treatment | flux ± SD µg/h/cm² | N | R_{sc}±SD 10³*s/cm | P_{sc}±SD 10⁻⁵*cm/s | F |
| none | 7.05±1.05 | 3 | 233.5±46 | 0.45±0.07 | 0.87±0.13 |
| propylene-glyc. | 8.11±1.00 | 3 | 200.0±25 | 0.55±0.06 | 1.00 |
| C2 | 9.20±1.60 | 4 | 169.0±34 | 0.59±0.12 | 1.05±0.20 |
| C6 | 10.6±0.25 | 3 | 142.0± 4 | 0.70±0.02 | 1.20±0.08 |
| C8 | 14.3±1.45 | 3 | 102.0±13 | 0.99±0.13 | 2.13±0.21 |
| C10 | 14.0±0.70 | 3 | 105.0± 7 | 0.96±0.06 | 2.13±0.18 |
| C12 | 11.3±0.50 | 3 | 133.0± 9 | 0.75±0.05 | 1.67±0.15 |
| C14 | 14.4±1.20 | 3 | 100.0±10 | 1.00±0.10 | 2.20±0.21 |
| C16 | 12.2±0.50 | 3 | 123.0± 6 | 0.81±0.04 | 1.80±0.15 |
| C18 | 18.1±3.30 | 3 | 74.0±18 | 1.35±0.32 | 3.00±0.51 |
| pretreatments were performed with 10 % enchancer in propyleneglycol, Cx denotes the length of the alkyl chain of the azone; C18 stands for oleyl. N = number of stratum corneum samples tested P_{sc} = the permeability coefficient through stratum corneum, calculated and corrected for the contribution of the silastic sheeting. P_{sc} = J/Cd, the steady state drug flux through the stratum corneum (=J), divided by the donor concentration (=Cd). R_{sc} = the resistance coefficient for stratum corneum, calculated and corrected for the contribution of the silastic sheeting, i.e. obtained by substracting the silastic sheeting contribution from the total resistance of the sandwich (= 1/P_{sc}). F = factor of comparison, the socalled 'Enhancement Factor'. | | | | | |

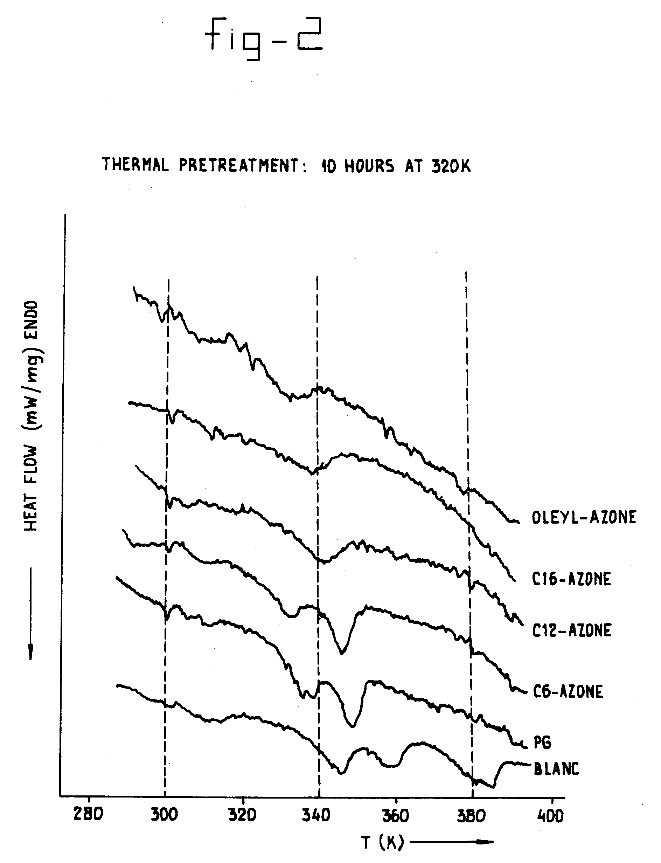

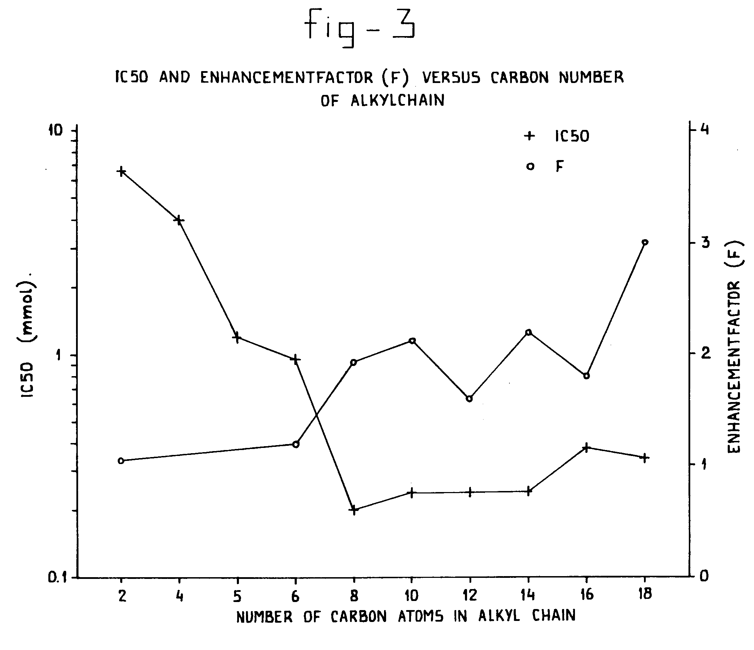

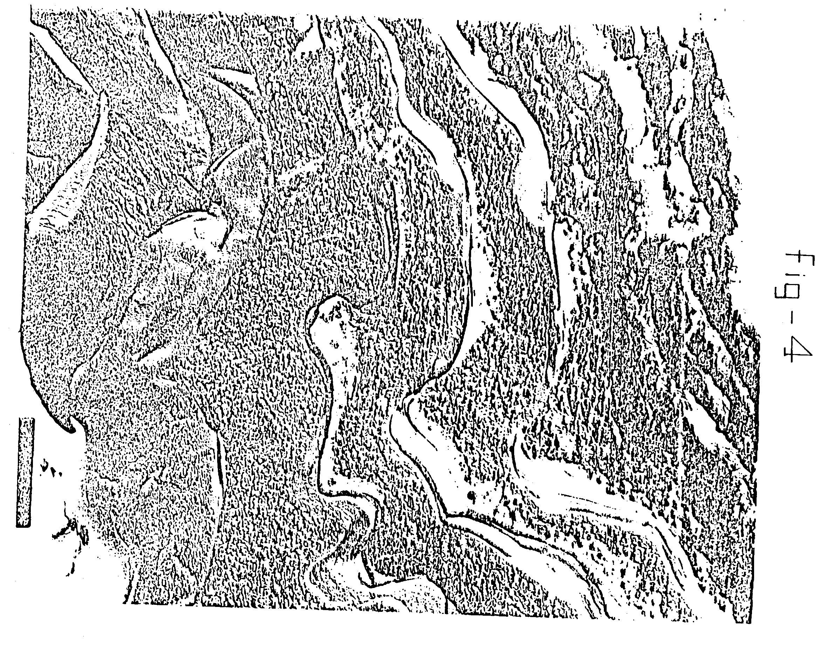

Re test 2:
The DTA results (fig. 2) show that oleyl cone strongly influences both the gel-liquid transition temperature of the intercellular lipids, and the corresponding transition enthalpy. (3.3 J.g-1 vs. 7 J.g-1 of the control). It is pointed out that the change in enthalpy is especially meaningful, and would correspond to a large degree of acyl chain disorder in the lipid bilayers in the intercellular space in the stratum corneum. This would then strongly enhance the overall permeability of the tissue.
Re test 3:
The electron microscopic results show, that especially the oleyl cone preparation penetrates deeply into the stratum corneum and induces the separation of a non-lamellar phase, which looks much less structured than the endogeneous lamellar lipid phase, and should therefore be more permeable (Fig. 4).
Re test 4:
The toxicity tests (Fig. 3) indicate that oleyl azone is almost the least toxic azone among the effective enhancers (from C10 up to C18), and less toxic than C12 azone (IC50 0.8 mM vs. 0.24 mM).

Summarized it can be stated that the compound (1-oleyl-azacycloheptan-2-one) is a new, effective, moderately toxic penetration enhancer.

## Claims

1. 1-Oleyl-azacycloheptan-2-one.

2. A composition comprising at least one physiologically active agent and an effective amount of 1-oleylazacycloheptan-2-one as a human or animal epithelial membrane permeability enhancing agent.

3. The composition according to claim 2, wherein the physiologically active agent is selected from the group consisting of antibacterial agents, antifungal agents, active steroids, retinoids, antihistaminic agents, radical scavengers, antihypertensives and other cardiovascular drugs, sex hormones, anti-diabetics, hypnotics, anti-parkinson drugs, local anaesthetics, anticoagulants, analgesics and antimigraine drugs.

4. The composition according to any one of the claims 1-3, wherein the 1-oleyl-azacycloheptan-2-one is present in a concentration ranging between 1-95 % by weight, preferably 5-40 % by weight.

5. Devices like (trans)dermal patches, bandages and dressings comprising a layer containing a composition according to any one of claims 2-4.

## Patentansprüche

1. 1-Oleylazacycloheptan-2-on.

2. Zusammensetzung, enthaltend mindestens einen physiologischen Wirkstoff und eine wirksame Menge an 1-Oleylazacycloheptan-2-on als Verstärkungsmittel für die Permeabilität einer menschlichen oder tierischen Epithelmembran.

3. Zusammensetzung nach Anspruch 2, wobei der physiologische Wirkstoff aus folgender Gruppe ausgewählt ist: antibakterielle Mittel, antimykotische Mittel, aktive Steroide, Retinoide, Antihistaminika, Radikalfänger, Antihypertensiva und andere kardiovaskuläre Arzneistoffe, Sexualhormone, Antidiabetika, Hypnotika, Antiparkinsonmittel, Lokalanästhetika, Antikoagulantien, Analgetika und Antimigränemittel.

4. Zusammensetzung nach einem der Ansprüche 1 bis 3, wobei das 1-Oleylazacycloheptan-2-on in einer Konzentration im Bereich von 1 bis 95 und vorzugsweise von 5 bis 40 Gew.-% vorhanden ist.

5. Vorrichtungen, wie (trans)dermale Pflaster, Binden und Verbände, umfassend eine Schicht mit einem Gehalt an einer Zusammensetzung nach einem der Ansprüche 2 bis 4.

## Revendications

1. 1-Oléyl-azacycloheptane-2-one.

2. Composition comprenant au moins un agent physiologiquement actif et une quantité efficace de 1-oléyl-azacycloheptane-2-one comme agent augmentant la perméabilité de la membrane épithéliale humaine ou animale.

3. Composition selon la revendication 2 dans laquelle l'agent physiologiquement actif est choisi dans le groupe constitué par les agents anti-microbiens, les agents antifongiques, les stéroïdes actifs, les rétinoïdes, les agents anti-histaminiques, les absorbeurs de radicaux, les antihypertenseurs et autres médicaments cardiovasculaires, les hormones sexuelles, les anti-diabétiques, les hypnotiques, les médicaments anti-parkinsoniens, les anesthésiques locaux, les anti-coagulants, les analgésiques et les médicaments anti-migraineux.

4. Composition selon l'une quelconque des revendications 1 à 3 dans laquelle la 1-oléyl-azacycloheptane-2-one est présente en une concentration comprise entre 1-95 % en poids, de préférence 5-40 % en poids.

5. Dispositifs tels que des patchs transdermiques, des bandages et des pansements comprenant une couche contenant une composition selon l'une quelconque des revendications 2 à 4.
